# EUROPEAN PATENT APPLICATION

(11) **EP 4 480 305 A1**
(43) Date of publication of application: **25.12.2024**
(21) Application number: 23774383.6
(22) Date of filing: 24.02.2023
(51) Int. Cl.: A01K 23/00

(54) **DIAPER FOR PETS**

(30) Priority: 24.03.2022 JP 2022048332
(71) Applicant: Unicharm Corporation, Ehime 799-0111 (JP)
(72) Inventor: KOIDO, Yumi, Kanonji-shi, Kagawa 769-1602 (JP); KOMATSUBARA, Daisuke, Kanonji-shi, Kagawa 769-1602 (JP)
(74) Representative: Dolleymores
(86) International application number: PCT/JP2023/006858
(87) International publication number: WO 2023/181784

(57) **Abstract**

In one aspect, a diaper for pets that is accommodated in a package in a folded state is provided. The diaper for pets has a longitudinal direction connecting a ventral side to a dorsal side of a pet and a width direction perpendicular to the longitudinal direction, and includes a main body portion including a front sheet, a back sheet, and an absorbent core disposed between the front sheet and the back sheet. A first cut portion to form a first opening through which a tail of a pet can be inserted and a second cut portion to form a second opening through which feces of the pet are capable of passing, the second opening having an area larger than an area of the first opening, and at least a part of the first cut portion and the second cut portion is disposed on an outer surface of the folded main body portion.

## Description

### Technical Field

The present disclosure relates to a diaper for pets.

### Background Art

A diaper for pets worn on a pet such as a dog or a cat is known. For example, Patent Literature 1 below describes a diaper for pets including a first cut portion that forms a first tail hole, a second cut portion that is disposed between the first cut portion and an absorbent core and forms a second tail hole, and an easily incised portion that extends between the first cut portion and the second cut portion. When the diaper for pets is worn on a male dog, the absorbent core is disposed in front of the base of the back leg by inserting the tail into the first tail hole. On the other hand, in a case where the diaper for pets is worn on a female dog, the absorbent core is disposed near the base of the back leg by inserting the tail into the second tail hole. As described above, in the diaper for pets described in Patent Literature 1, by selecting the tail hole through which the tail passes according to the sex of the dog, the absorbent core is disposed at a position covering the urine outlet of the dog, and thus urine leakage is reduced.

### Citation List

### Patent Literature

Patent Literature 1: Japanese Unexamined Patent Publication No. 2012-205542

### Summary of Invention

### Technical Problem

By the way, whether to discharge the feces (feces) of the pet to an inside or an outside of the diaper varies depending on preference or a situation of a user. For example, when the pet is in the room, it may be preferable to cause the pet to defecate inside of the diaper so that the room is not dirty, and when the pet is outside the room, it may be preferable to cause the pet to defecate outside of the diaper so that the feces do not adhere to the pet's hair.

Therefore, it is conceivable to form two types of openings having different sizes in the diaper for pets so that it is possible to select whether to cause to defecate inside or outside of the diaper. However, even when the two types of openings are formed in the diaper for pets, it is not possible to cause the user to recognize that the user can select an evacuation destination unless the user notices the presence of these openings.

Therefore, an object of the present disclosure is to make it easy for a user to recognize that it is possible to select whether to cause to defecate inside or outside of a diaper.

### Solution to Problem

In one aspect, a diaper for pets that is accommodated in a package in a folded state is provided. The diaper for pets includes a main body portion having a longitudinal direction connecting a ventral side to a dorsal side of a pet and a width direction perpendicular to the longitudinal direction, the main body portion including a front sheet, a back sheet, and an absorbent core disposed between the front sheet and the back sheet, wherein the main body portion is formed with a first cut portion to form a first opening through which a tail of the pet is capable of being inserted, and a second cut portion to form a second opening through which feces of the pet are capable of passing, the second opening having an area larger than an area of the first opening, and at least a part of the first cut portion and the second cut portion is disposed on an outer surface of the folded main body portion.

In the diaper for pets of the present aspect, at least a part of the first cut portion forming the first opening through which the tail of the pet can be inserted and the second cut portion forming the second opening through which the feces of the pet can pass is disposed on the outer surface of the folded main body portion. Therefore, when the diaper for pets is taken out from the package, the user can visually recognize the first cut portion and the second cut portion. As a result, it is possible to cause the user to recognize that it is possible to select whether to cause to defecate inside or outside of the diaper.

In one embodiment, the diaper for pets may be folded in a state of being folded in three or four in the longitudinal direction, and entire the first cut portion and the second cut portion may be disposed on the outer surface of the folded main body portion. Since the entire the first cut portion and the second cut portion are disposed on the outer surface of the folded main body portion, it is possible to make it easy for the user to recognize that the user can select the evacuation destination.

In one embodiment, the main body portion may include a non-cut portion disposed between the first cut portion and the second cut portion, and the second cut portion may form the second opening enclosing the first opening together with the first cut portion when the non-cut portion is broken and the first cut portion and the second cut portion are coupled. In this embodiment, when the non-cut portion is broken, the second opening is formed, and the feces of the pet are discharged to the outside of the diaper. On the other hand, when the non-cut portion is not broken, since the second opening is not formed, the feces of the pet are discharged to the inside of the diaper. Therefore, it is possible to select whether to cause to defecate inside or outside of the diaper.

In one embodiment, the first cut portion may be formed on the dorsal side of the main body portion with respect to the absorbent core, and the second cut portion may be formed on the dorsal side of the main body portion with respect to the first cut portion. In this embodiment, by breaking the non-cut portion, an opening formed in the main body portion can be enlarged to the dorsal side.

In one embodiment, the first cut portion may be configured to form a flap-shaped tongue piece that is a part of the front sheet and the back sheet, and when the tongue piece is pulled to the dorsal side, the non-cut portion may be broken to couple the first cut portion and the second cut portion. In this embodiment, the second opening can be easily formed by pulling the tongue piece to the dorsal side. In addition, since the second opening is formed by pulling the tongue piece in a direction opposite to the absorbent core, the absorbent core is prevented from being accidentally broken when the non-cut portion is broken.

In one embodiment, the first cut portion may include a U-shaped slit protruding toward the ventral side, and the second cut portion may include a pair of slits adj acent to a pair of end portions of the U-shaped slit via the non-cut portion. In this embodiment, the pair of end portions of the U-shaped slit are coupled to the pair of slits by breaking the non-cut portion, and the second opening is formed.

In one embodiment, the pair of slits may linearly extend so as to be separated from each other toward the dorsal side. In this embodiment, when the tongue piece is pulled to the dorsal side to break the non-cut portion, a force is dispersed in a width direction of the diaper for pets, so that the main body portion is hardly torn to the dorsal side accidentally when the second opening is formed.

In one embodiment, the first cut portion may include an annular slit extending over an entire periphery of an end edge of the first opening. By having the annular slit, a part of the front sheet and the back sheet is cut out, so that it is possible to cause the user to easily recognize the presence of the first opening.

In one embodiment, the first cut portion may be formed on the dorsal side of the main body portion with respect to the absorbent core, and the second cut portion may be formed between the first cut portion and the absorbent core in the longitudinal direction. In this embodiment, when the second opening is formed by breaking the non-cut portion, the opening expands toward the ventral side, so that a position of the dorsal side end edge of the opening does not change. That is, since there is no change in a positional relationship between the tail of the pet and the diaper for pets, displacement is less likely to occur in the diaper for pets. As a result, urine leakage can be suppressed.

In one embodiment, the first cut portion may be configured to form a flap-shaped tongue piece that is a part of the front sheet and the back sheet, and when the tongue piece is pulled to the ventral side, the non-cut portion may be broken to couple the first cut portion and the second cut portion. In this embodiment, by pulling the tongue piece to the ventral side, the non-cut portion can be easily cut to form the second opening.

In one embodiment, the first cut portion may include a U-shaped slit protruding toward the dorsal side, and the second cut portion may include a pair of slits adjacent to a pair of end portions of the U-shaped slit via the non-cut portion. In this embodiment, the pair of end portions of the U-shaped slit are coupled to the pair of slits by cutting the non-cut portion, and the second opening is formed.

In one embodiment, the pair of slits may linearly extend so as to be separated from each other toward the ventral side. In this embodiment, since the force is dispersed in the width direction of the diaper for pets when the tongue piece is pulled to the ventral side to break the non-cut portion, it is possible to prevent the absorbent core from being accidentally broken when the second opening is formed.

In one embodiment, the first cut portion may include an annular slit extending over an entire periphery of an end edge of the first opening. By having the annular slit, a part of the front sheet and the back sheet is cut out, so that it is possible to cause the user to easily recognize the presence of the first opening.

The diaper for pets according to one embodiment may further include a pair of fastening tabs extending outward of the main body portion in the width direction, and a target portion that is disposed on the dorsal side of the main body portion with respect to the pair of fastening tabs and engaged with the pair of fastening tabs, and a dorsal side end edge of the second opening may be disposed on the ventral side with respect to an intermediate line between a center line of the main body portion and a ventral side end edge of the target portion in the longitudinal direction. By disposing the dorsal side end edge of the second opening at such a position, the dorsal side end edge of the second opening can be reliably abutted against the back of the tail of the pet when the diaper for pets is worn. Thus, the displacement of the diaper for pets is suppressed. As a result, it is possible to cause to discharge the feces to the outside of the diaper for pets while preventing urine leakage.

In one embodiment, an area of the second opening may be twice or more an area of the first opening. By setting the area of the second opening to be twice or more the area of the first opening, the feces of the pet can be reliably discharged to the outside of the diaper for pets.

In one embodiment, a maximum length of the second opening in the longitudinal direction may be twice or more a maximum length of the first opening in the longitudinal direction. By setting the length of the second opening in the longitudinal direction to twice or more the length of the first opening in the longitudinal direction, it is possible to reliably discharge the feces of the pet to the outside of the diaper for pets.

In one embodiment, a maximum width of the first opening in the width direction may be larger than the maximum length of the first opening in the longitudinal direction. By forming the first opening into a horizontally long shape, the tail of the pet can easily pass through the first opening.

In one embodiment, a maximum length of the second opening in the longitudinal direction may be larger than a maximum width of the second opening in the width direction. By forming the second opening into a vertically long shape that is long in the longitudinal direction, the feces of the pet can be easily passed.

The diaper for pets of one embodiment may further include an elastic member fixed to the main body portion in a state of being stretched in the longitudinal direction and extending so as to straddle the second opening in the longitudinal direction. When the elastic member contracts in the longitudinal direction, a region between the second opening of the main body portion and the absorbent core is pulled and pressed against buttocks of the pet. As a result, a fit of the diaper for pets is improved, and a gap is less likely to occur between the end edge of the second opening and the buttocks of the pet. Therefore, the feces of the pet can be reliably discharged to the outside of the diaper for pets.

In one embodiment, a design may be imparted to a surface of the back sheet, and a design may not be imparted to a surface of the front sheet. In this embodiment, since the front sheet to which the design is not imparted becomes visible through the first opening, it becomes easy for the user to visually recognize the presence of the first opening from a difference in contrast.

### Advantageous Effects of Invention

According to various aspects and embodiments of the present invention, it is possible to make it easy for a user to recognize that it is possible to select whether to cause to defecate inside or outside of the diaper.

### Brief Description of Drawings

FIG. 1 is a plan view illustrating a diaper for pets according to one embodiment.
FIG. 2 is a cross-sectional view taken along line II-II of FIG. 1.
FIG. 3 is an enlarged view of a periphery of a first cut portion and a second cut portion.
FIG. 4 is a view illustrating a state in which the diaper for pets is worn by passing a tail through a first opening.
FIG. 5 is an enlarged view of the periphery of the first cut portion and the second cut portion.
FIG. 6 is a view illustrating a state in which the diaper for pets is worn by passing the tail through a second opening.
FIG. 7 is a view illustrating the diaper for pets accommodated in a package in a folded state.
FIG. 8 is a plan view illustrating an example of the folded diaper for pets.
FIG. 9 is a plan view illustrating a modification of the diaper for pets.
FIG. 10 is a plan view illustrating the modification of the diaper for pets.
FIG. 11 is a plan view illustrating another modification of the diaper for pets.
FIG. 12 is a plan view illustrating another modification of the diaper for pets.
FIG. 13 is a plan view illustrating another modification of the diaper for pets.

### Description of Embodiments

Hereinafter, an embodiment of the present disclosure will be described with reference to the drawings. In the following description, the same or equivalent elements are denoted by the same reference numerals, and redundant description will not be repeated. The dimensional ratios in the drawings do not necessarily coincide with those in the description.

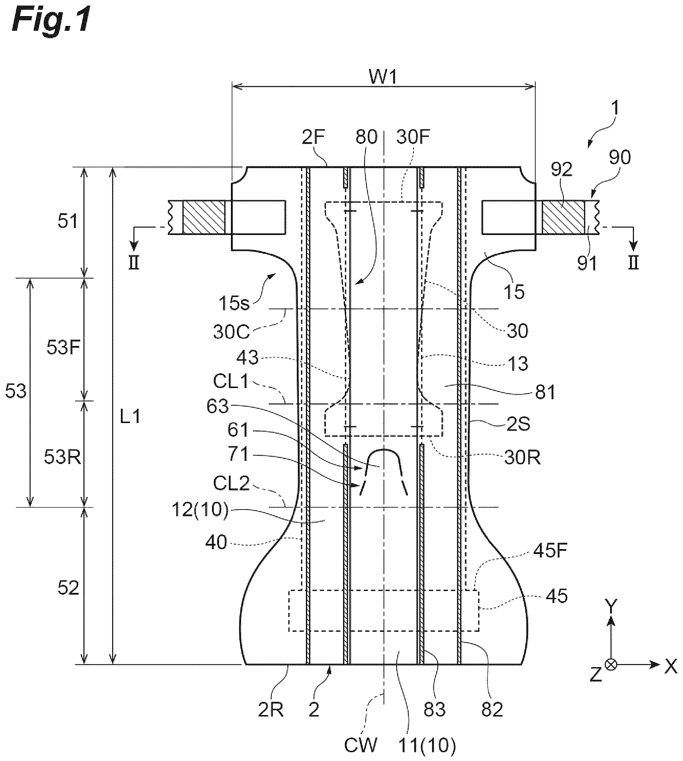

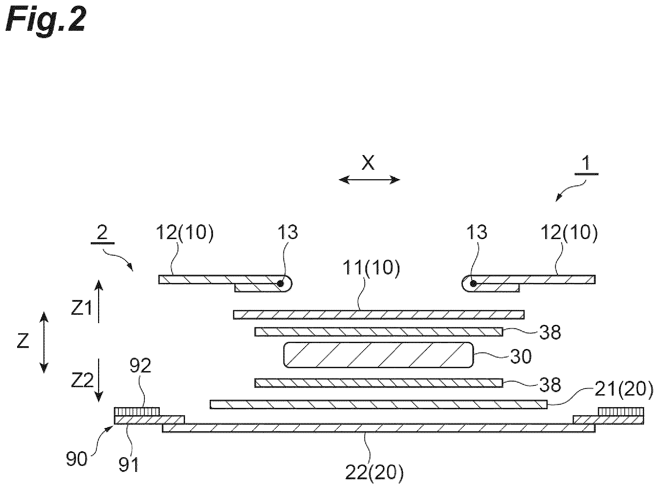

FIG. 1 is a plan view of a diaper for pets according to one embodiment as viewed from a skin surface side Z1. FIG. 2 is a cross-sectional view schematically illustrating the diaper for pets taken along line II-II illustrated in FIG. 1. FIG. 1 illustrates a diaper for pets 1 in a stretched state in which the diaper for pets 1 is stretched until no wrinkle is formed. In the following description, a positional relationship in the stretched state will be described unless otherwise specified. In the cross-sectional view illustrated in FIG. 2, for convenience of explanation, members are illustrated spaced apart in a thickness direction Z, but in an actual product, the members are in contact with each other in the thickness direction Z.

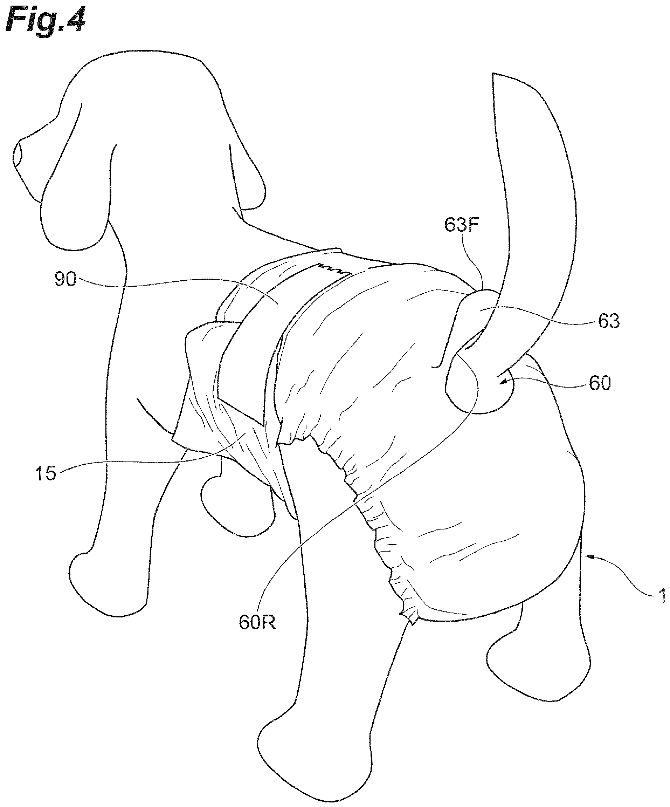

The diaper for pets 1 is a diaper used for pets. In the present specification, the term "pet" broadly encompasses vertebrates and invertebrates, and typically includes pets such as dogs, cats, rabbits, and hamsters. The diaper for pets 1 of one embodiment is particularly suitably used as a diaper for small animals such as a puppy, a small dog, and a cat. As illustrated in FIG. 1, the diaper for pets 1 extends in a width direction X disposed along a waistline direction of a pet and in a longitudinal direction Y connecting a ventral side to a dorsal side of the pet. The width direction X and the longitudinal direction Y are directions perpendicular to each other. The thickness direction Z is a direction perpendicular to the width direction X and the longitudinal direction Y, and includes a skin surface side Z1 facing the skin surface of the pet in a wearing state and a non-skin surface side Z2 facing an opposite side to the skin surface of the pet in the wearing state. As illustrated in FIG. 4, the diaper for pets 1 is worn so as to cover from the ventral side to the dorsal side through a crotch of the pet.

The diaper for pets 1 has a main body portion 2. The main body portion 2 extends in the width direction X and the longitudinal direction Y In the following description, one end side in the longitudinal direction Y of the main body portion 2 may be referred to as a ventral side, and the other end side in the longitudinal direction Y may be referred to as a dorsal side. As illustrated in FIGS. 1 and 2, the main body portion 2 includes a front sheet 10, a back sheet 20, and an absorbent core 30. The front sheet 10 constitutes a surface of the main body portion 2 that is brought into contact with a pet, and is disposed on the skin surface side Z1. The front sheet 10 has liquid permeability that allows urine of a pet to permeate toward the absorbent core 30. The front sheet 10 is located at the center in the width direction X and includes a center sheet 11 covering the absorbent core 30 and a pair of side sheets 12 covering both side portions of the center sheet 11 in the width direction X. For example, the center sheet 11 and the pair of side sheets are formed of a plain nonwoven fabric to which a design is not imparted.

As illustrated in FIG. 2, inner portions of the pair of side sheets 12 in the width direction X are folded back to the non-skin surface side Z2. A pair of elastic members 13 in a state of being stretched in the longitudinal direction Y are disposed between the pair of folded side sheets 12. The pair of side sheets 12 and the pair of elastic members 13 constitute a leakage-preventing gather 80. The leakage-preventing gather 80 is an upright leakage-preventing gather and is disposed on the skin surface side Z1 than the absorbent core 30.

The leakage-preventing gather 80 is disposed outside the absorbent core 30 in the width direction X, and prevents urine excreted to the absorbent core 30 from leaking in the width direction X. In one embodiment, the leakage-preventing gather 80 includes a pair of elastic members 13 extending in the longitudinal direction Y, an upright portion 81 that stands up due to contraction of the pair of elastic members 13, a lateral fixing portion 82 that serves as an upright fulcrum of the upright portion 81 in the width direction X, and a vertical fixing portion 83 that serves as an upright fulcrum of the upright portion 81 in the longitudinal direction Y

The pair of elastic members 13 are provided in inner portions of the side sheets 12 in the width direction X and extend in the longitudinal direction Y The pair of elastic members 13 are made of, for example, a stretchable material such as rubber or spandex that can be contracted in a length direction. The pair of elastic members 13 are disposed apart from each other with a center line CW in the width direction X of the main body portion 2 therebetween, and are fixed to the side sheets 12 in a state of being stretched in the longitudinal direction Y Ventral side end portions of the pair of elastic members 13 are disposed in a ventral region 51 to be described later, and dorsal side end portions of the pair of elastic members 13 are disposed in a buttock region 53R to be described later.

The lateral fixing portion 82 is formed in substantially the entire region of the main body portion 2 in the longitudinal direction Y at a position outside the upright portion 81 in the width direction X in the side sheet 12. That is, the lateral fixing portion 82 is disposed outside the absorbent core 30 in the width direction X. The lateral fixing portion 82 is a part of the side sheet 12, and is fixed to the center sheet 11. The lateral fixing portion 82 serves as an upright fulcrum of the upright portion 81 in the width direction X.

The vertical fixing portions 83 are formed on each of the ventral side and the dorsal side of the main body portion 2 at positions on the inner side in the width direction X with respect to the lateral fixing portion 82. The vertical fixing portion 83 is a part of the side sheet 12 and is fixed to the center sheet 11. The vertical fixing portion 83 serves as an upright fulcrum of the upright portion 81 in the longitudinal direction Y

The upright portion 81 is formed between the vertical fixing portions 83 formed on the ventral side and the dorsal side of the main body portion 2, respectively. That is, the upright portion 81 is disposed at a position on the inner side in the width direction X than the lateral fixing portion 82 and at a position on the inner side in the longitudinal direction Y than the vertical fixing portion 83. The upright portion 81 is a portion of the side sheet 12 that is not fixed to the center sheet 11. The elastic member 13 stretched in the longitudinal direction Y is coupled to the upright portion 81. When the elastic member 13 contracts in the longitudinal direction Y, the main body portion 2 is deformed in the closing direction, and the upright portion 81 rises from the lateral fixing portion 82 as a starting point. Urine leakage is prevented by the upright portion 81 standing up at the position where the absorbent core 30 is sandwiched from the width direction X in this manner.

The back sheet 20 constitutes a surface located on the outer side when worn in the main body portion 2, and is disposed on the non-skin surface side Z2. The back sheet 20 includes a liquid-impermeable back surface film 21 and a back surface nonwoven fabric 22 located on the non-skin surface side Z2 than the back surface film 21. A length of the back surface film 21 in the width direction X may be formed shorter than a length of the back surface nonwoven fabric 22 in the width direction X, and the back surface nonwoven fabric 22 may extend to both sides in the width direction X than the back surface film 21. The back surface nonwoven fabric 22 constitutes an outermost layer of the diaper for pets 1. A design may be imparted to a front surface of the back surface nonwoven fabric 22 on the non-skin surface side Z2.

The absorbent core 30 is disposed between the front sheet 10 and the back sheet 20. The absorbent core 30 includes, for example, a plant-derived pulp fiber having water absorbency and a polymer absorbent (SAP), and absorbs urine of pets. As illustrated in FIG. 2, an upper surface and a lower surface of the absorbent core 30 may be covered with core wraps 38. The absorbent core 30 is disposed substantially at the center of the main body portion 2 in the width direction X.

The absorbent core 30 has a ventral side end edge 30F and a dorsal side end edge 30R. A width of the ventral side end edge 30F and the dorsal side end edge 30R in the width direction X is larger than a distance between the pair of elastic members 13 in the width direction X. A constricted portion 43 in which the width of the absorbent core 30 is narrowed is formed between the ventral side end edge 30F and the dorsal side end edge 30R. A width of the constricted portion 43 in the width direction X is smaller than the distance between the pair of elastic members 13 in the width direction X.

The constricted portion 43 is disposed at a position on the ventral side than a center line CL1 of the main body portion 2 in the longitudinal direction Y and at a position on the dorsal side than a center line 30C of the absorbent core 30 in the longitudinal direction Y Note that the center line CL1 is an imaginary line located at an equal distance from a dorsal side end edge 2R and a ventral side end edge 2F of the main body portion 2 described later and extending along the width direction X. That is, the center line CL1 is a portion where a fold is formed when the diaper for pets 1 is folded in half in the longitudinal direction Y

The dorsal side end edge 30R of the absorbent core 30 is disposed on the dorsal side than the center line CL1 of the main body portion 2 in the longitudinal direction Y The ventral side end edge 30F of the absorbent core 30 is disposed on the ventral side than the center line CL1. When the diaper for pets 1 is worn on the pet, the pet's urination port is brought into contact with a region near the center line CL1 of the main body portion 2.

The main body portion 2 has the ventral side end edge 2F located on the ventral side in the longitudinal direction Y and the dorsal side end edge 2R located on the dorsal side in the longitudinal direction Y The ventral side end edge 2F is disposed in the waistline direction of the pet on the ventral side when worn, and the dorsal side end edge 2R is disposed in the waistline direction of the pet on the dorsal side when worn. A distance between the ventral side end edge 2F and the dorsal side end edge 2R in the longitudinal direction Y is a total length (maximum length) L1 of the main body portion 2. A maximum width W1 of the main body portion 2 in the width direction X may be larger than half of the total length L1 of the main body portion 2 in the longitudinal direction.

In addition, the main body portion 2 has a ventral region 51 located on the ventral side end edge 2F side, a dorsal region 52 located on the dorsal side end edge 2R side, and an intermediate region 53 located between the ventral region 51 and the dorsal region 52 in the longitudinal direction Y A width of the intermediate region 53 in the width direction X is substantially constant in the longitudinal direction Y Widths of the ventral region 51 and the dorsal region 52 in the width direction X may be larger than the width of the intermediate region 53 in the width direction X.

The intermediate region 53 includes a crotch region 53F located between the center line CL1 of the main body portion 2 and the ventral region 51, and the buttock region 53R located between the center line CL1 of the main body portion 2 and the dorsal region 52. The crotch region 53F is a region that covers the crotch of the pet when the diaper for pets 1 is worn on the pet. The buttock region 53R is a region that abuts on the buttocks of the pet when the diaper for pets 1 is worn on the pet.

A pair of flap portions 15 extending outward in the width direction X from the main body portion 2 are formed in the dorsal region 52 of the main body portion 2. The pair of flap portions 15 define a pair of leg surrounding openings 15s through which the rear legs of the pet pass. A width between side end edges of the pair of flap portions 15 in the width direction X corresponds to the maximum width W1 of the main body portion 2.

Each of the pair of flap portions 15 is provided with a pair of fastening tabs 90. The pair of fastening tabs 90 have a rectangular shape that is long in the width direction X when viewed from the skin surface side Z1, and are disposed close to the ventral side end edge 2F of the main body portion 2 in the longitudinal direction Y The pair of fastening tabs 90 extend outward in the width direction X from side end edges 2S of the main body portion 2. Each of the pair of fastening tabs 90 includes a base material sheet 91 joined to the flap portion 15 and a joining portion 92 provided on the base material sheet 91. The joining portion 92 is disposed on the skin surface side Z1 surface of the fastening tab 90. The joining portion 92 is, for example, a mechanical fastener, and is configured to be able to be joined to a target portion 45 formed on the non-skin surface side Z2 of the main body portion 2.

The target portion 45 is provided on the non-skin surface side Z2 of the dorsal region 52 of the main body portion 2. As illustrated in FIG. 1, the target portion 45 has a belt-like shape extending in the width direction X. The target portion 45 is disposed on the dorsal side end edge 2R side than the pair of fastening tabs 90, and engaged with the pair of fastening tabs 90. The main body portion 2 may not include the target portion 45, and may be configured such that the joining portions 92 of the pair of fastening tabs 90 are directly joined to the back surface nonwoven fabric 22 of the back sheet 20.

A first cut portion 61 and a second cut portion 71 are formed in the main body portion 2. The first cut portion 61 forms a first opening 60 through which the tail of the pet is inserted. The second cut portion 71 forms a second opening 70 through which the feces of the pet pass. As illustrated in FIG. 1, the first cut portion 61 and the second cut portion 71 are disposed on the ventral side than an intermediate line CL2 between the center line CL1 of the main body portion 2 in the longitudinal direction Y and a ventral side end edge 45F of the target portion 45. That is, the first cut portion 61 and the second cut portion 71 are formed in the buttock region 53R of the main body portion 2.

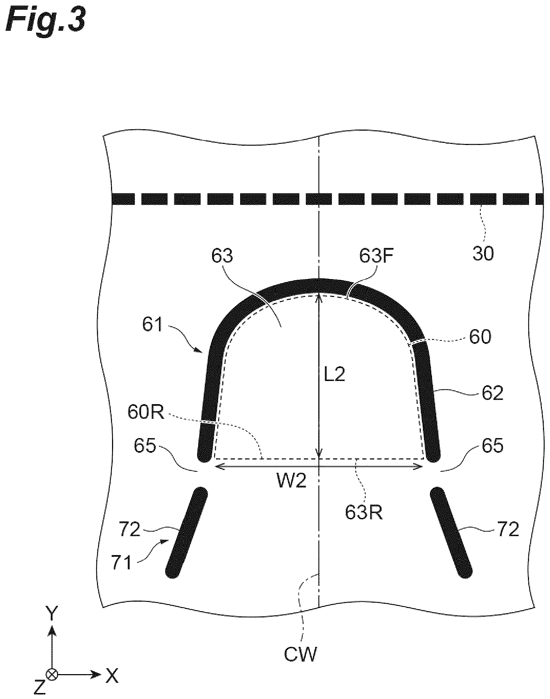

FIG. 3 is an enlarged view of the periphery of the first cut portion 61 and the second cut portion 71. As illustrated in FIG. 3, the first cut portion 61 is formed on the dorsal side of the main body portion 2 than the absorbent core 30. The second cut portion 71 is formed on the dorsal side of the main body portion 2 than the first cut portion 61. That is, the first cut portion 61 is formed between the absorbent core 30 and the second cut portion 71 in the longitudinal direction Y A non-cut portion 65 in which a slit is not formed is provided between the first cut portion 61 and the second cut portion 71. That is, the first cut portion 61 and the second cut portion 71 are spaced apart from each other via the non-cut portion 65.

As illustrated in FIG. 3, the first cut portion 61 includes a slit 62 having a substantially U-shape protruding toward the absorbent core 30 as viewed from the skin surface side Z1. The slit 62 is disposed on the center line CW in the width direction X and penetrates the front sheet 10 and the back sheet 20 of the main body portion 2 in the thickness direction. Note that the center line CW is an imaginary line located at an equal distance from the pair of side end edges 2S of the main body portion 2 in the width direction X and extending along the longitudinal direction Y

The main body portion 2 has a flap-shaped tongue piece 63. The tongue piece 63 is a portion formed by making a substantially U-shaped cut in the main body portion 2 by the slit 62, and is configured by a part of the front sheet 10 and the back sheet 20. The tongue piece 63 has a base end portion 63R coupled to the buttock region 53R of the main body portion 2 and a distal end portion 63F disposed on the opposite side of the base end portion 63R and separated from the buttock region 53R. The distal end portion 63F is directed toward the ventral side in a state where the tongue piece 63 is not folded back.

The tongue piece 63 is folded back toward the non-skin surface side Z2 starting from the base end portion 63R to open the first opening 60. The first opening 60 is an opening having a semicircular shape or a tongue shape, and functions as a tail hole through which the tail of the pet is inserted. Since the first opening 60 is located on the dorsal side than the dorsal side end edge 30R of the absorbent core 30, regions where the absorbent core 30 is not disposed are formed on the outer side and the dorsal side of the first opening 60 in the width direction X.

FIG. 4 illustrates a state in which the diaper for pets is worn by passing the tail through the first opening 60. Since the distal end portion 63F of the tongue piece 63 is directed toward the ventral side, as illustrated in FIG. 4, when the tongue piece 63 is folded back toward the non-skin surface side Z2 starting from the base end portion 63R by passing the tail through the first opening 60, the tongue piece 63 abuts on the back side of the tail. At this time, the dorsal side end edge 60R of the first opening 60 abuts on the tail of the pet, whereby the diaper for pets 1 is positioned.

In one embodiment, as illustrated in FIG. 3, a maximum width W2 of the first opening 60 in the width direction X is larger than a maximum length L2 of the first opening 60 in the longitudinal direction Y That is, the first opening 60 has a laterally long shape that is long in the width direction X. Therefore, as illustrated in FIG. 4, when the tail is passed through the first opening 60, an anus of the pet is not exposed from the first opening 60. Therefore, when the pet defecates while wearing the diaper for pets 1, the feces do not pass through the first opening 60 and are excreted inside of the diaper for pets 1.

The second cut portion 71 includes a pair of slits 72. In the embodiment illustrated in FIG. 3, the pair of slits 72 are spaced apart from each other and extend obliquely with respect to the center line CW. More specifically, the pair of slits 72 linearly extend so as to separate from each other as they separate from the absorbent core 30. Ventral side end portions of the pair of slits 72 are disposed adjacent to the pair of end portions of the substantially U-shaped slit 62. The non-cut portion 65 is formed between the ventral side end portions of the pair of slits 72 and the pair of end portions of the slit 62. Lengths of the pair of slits 72 may be longer than lengths of the pair of non-cut portions 65.

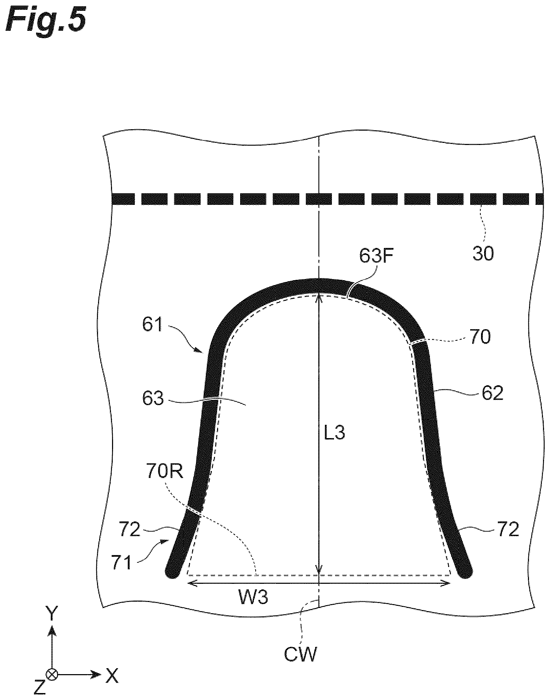

When the non-cut portion 65 is broken, as illustrated in FIG. 5, the slit 62 of the first cut portion 61 and the pair of slits 72 of the second cut portion 71 are coupled, and a substantially U-shaped slit longer than the slit 62 is formed. The non-cut portion 65 is easily broken by pinching the tongue piece 63 with fingers from the non-skin surface side Z2 of the diaper for pets 1 and pulling the tongue piece to the dorsal side. Accordingly, the tongue piece 63 is connected to a portion between the pair of slits 72 of the main body portion 2. In a state where the first cut portion 61 and the second cut portion 71 are coupled, the tongue piece 63 is folded back toward the non-skin surface side Z2, whereby the second opening 70 is opened. That is, the second opening 70 forms the second opening 70 together with the first cut portion 61 when the non-cut portion 65 is broken.

As illustrated in FIG. 5, the second opening 70 is an opening that encloses the first opening 60. That is, when the non-cut portion 65 is cut and the first cut portion 61 and the second cut portion 71 are coupled, the first opening 60 is enlarged to the dorsal side to form the second opening 70. The second opening 70 is an opening having a semicircular shape or a tongue shape, and functions as an enlarged tail hole. An area of the second opening 70 is larger than an area of the first opening 60, and can pass the feces of the pet. In one embodiment, the area of the second opening 70 may be twice or more the area of the first opening 60. In the embodiment illustrated in FIG. 5, an opening width of the second opening 70 in the width direction X increases toward the dorsal side.

In order to reliably pass the feces of the pet, the second opening 70 may have a vertically long shape that is long in the longitudinal direction Y For example, a maximum length L3 of the second opening 70 in the longitudinal direction Y is larger than a maximum width W3 of the second opening 70 in the width direction X. The maximum length L3 of the second opening 70 may be twice or more the maximum length L2 of the first opening 60 in the longitudinal direction Y

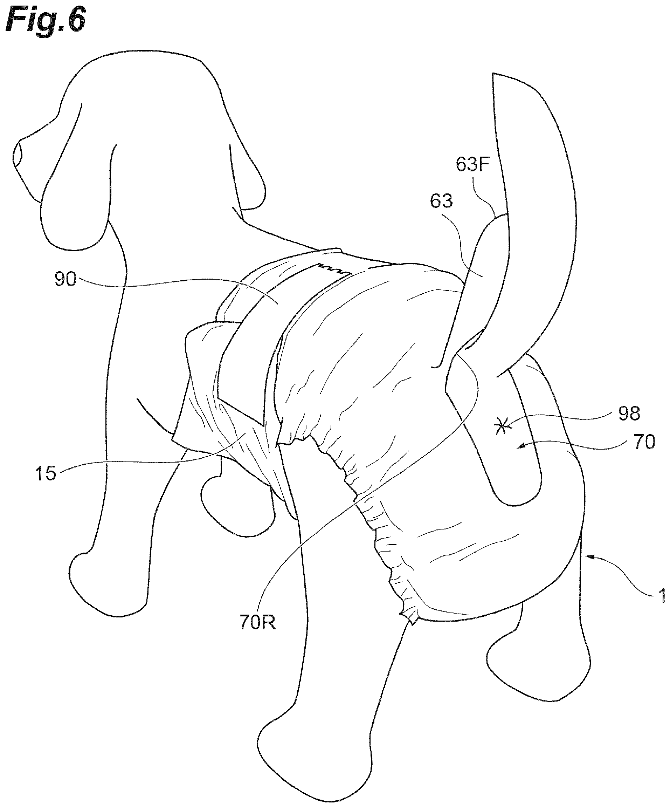

FIG. 6 is a view illustrating a state in which the diaper for pets 1 is worn on the pet by passing the tail through the second opening 70. As illustrated in FIG. 6, since the second opening 70 has a vertically long shape, when the tongue piece 63 is folded back toward the non-skin surface side Z2 by passing the tail through the second opening 70, an anus 98 of the pet is exposed from the second opening 70. Therefore, when the pet defecates while wearing the diaper for pets 1, the feces pass through the second opening 70 and are discharged to the outside of the diaper for pets 1. In a case where the diaper for pets 1 is worn on the pet by passing the tail through the second opening 70, a dorsal side end edge 70R of the second opening 70 abuts on the tail of the pet. Therefore, the position where the tail of the pet and the diaper for pets 1 abut is moved to the dorsal side as compared with the case where the diaper for pets 1 is worn on the pet by passing the tail through the first opening 60.

As described above, the user of the diaper for pets 1 can select whether to cause to defecate inside or outside of the diaper for pets 1. That is, when it is desired to defecate inside of the diaper for pets 1, the tail of the pet is inserted into the first opening 60 without cutting the non-cut portion 65. Since the first opening 60 has a size that does not pass through the feces, in this case, the feces of the pet are excreted inside of the diaper for pets 1. On the other hand, in a case where it is desired to cause to discharge the feces of the pet to the outside of the diaper for pets 1, the non-cut portion 65 is cut, and the tail of the pet is inserted into the second opening 70. Since the second opening 70 has a size capable of passing the feces, in this case, the feces of the pet are excreted outside of the diaper for pets 1.

As illustrated in FIG. 1, the diaper for pets 1 may further include a pair of elastic members 40 stretchable in the length direction. The pair of elastic members 40 are disposed outside in the width direction X than the elastic members 13. In the embodiment illustrated in FIG. 1, the ventral side end portions of the pair of elastic members 40 are disposed in the ventral region 51, and dorsal side end portions of the pair of elastic members 40 are disposed in the dorsal region 52. The pair of elastic members 40 are made of, for example, a stretchable material such as rubber or spandex that can be contracted in the length direction, and are disposed along the side end edges 2S of the main body portion 2 between the front sheet 10 and the back sheet 20 of the main body portion 2. Note that the pair of elastic members 40 may extend in the longitudinal direction Y in a state of being sewn to the front sheet 10 and the back sheet 20 of the main body portion 2. When the diaper for pets 1 is worn, the pair of elastic members 40 contract in the longitudinal direction Y, whereby the side end edges 2S of the main body portion 2 fit around the legs of the wearer.

A method of wearing the diaper for pets 1 will be described with reference to FIG. 4 or FIG. 6. Before the diaper for pets 1 is worn on the pet, first, the first opening 60 or the second opening 70 is formed depending on whether it is desired to cause to defecate inside or outside of the diaper for pets 1. Then, the tail of the pet is passed through the first opening 60 or the second opening 70 to bring the tail of the pet out to the non-skin surface side Z2 of the diaper for pets 1. At this time, the tongue piece 63 is folded back toward the non-skin surface side Z2, and the tongue piece 63 is disposed on the back side of the tail. Next, the ventral side end edge 2F of the main body portion 2 is brought into contact with the belly of the pet. Then, while the vicinity of the center line CL1 of the main body portion 2 in the longitudinal direction Y is brought into contact with the urination port of the pet, the dorsal side end edge 2R of the main body portion 2 is brought into contact with the back of the pet. Next, the pair of fastening tabs 90 are pulled to the back of the pet in the waistline direction, and the pair of fastening tabs 90 are fastened to the outer surface of the target portion 45 of the main body portion 2. As a result, as illustrated in FIG. 4 or 6, the diaper for pets 1 is worn so as to cover the belly, the back, and the crotch of the pet.

In a case where the tail of the pet is inserted into the first opening 60 without breaking the non-cut portion 65, as illustrated in FIG. 4, the anus of the pet is not exposed from the diaper for pets 1, and the feces of the pet are excreted inside of the diaper for pets 1. On the other hand, in a case where the non-cut portion 65 is broken and the tail of the pet is inserted into the second opening 70, as illustrated in FIG. 6, the anus 98 of the pet is exposed from the diaper for pets 1, and the feces of the pet are excreted outside of the diaper for pets 1.

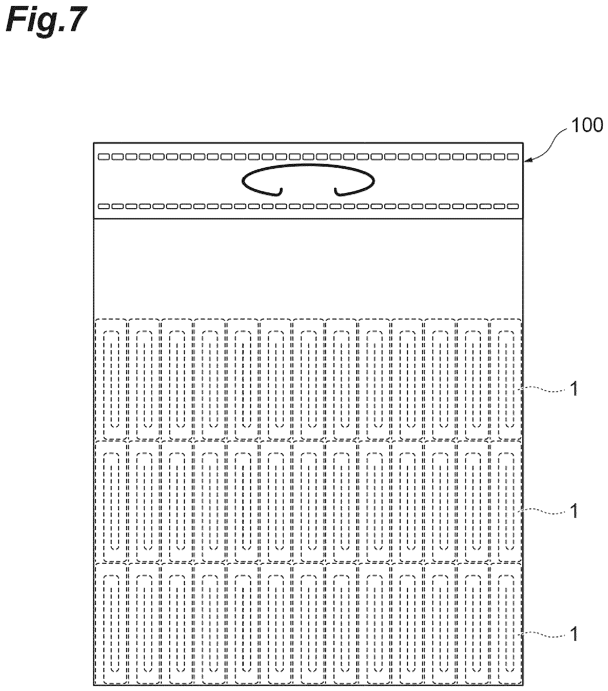

As illustrated in FIG. 7, the above-described diaper for pets 1 is accommodated in the package 100 in a folded state. For example, after the side sheet 12 is folded back inward in the width direction X, the diaper for pets 1 is folded in three or four in the longitudinal direction Y and accommodated in the package 100. In the embodiment illustrated in FIG. 7, a plurality of folded diapers for pets 1 are stored in the package 100 in a state of being stacked in the width direction and the vertical direction of the package 100.

An example of how to fold the diaper for pets 1 will be described. When the diaper for pets 1 is accommodated in the package 100, first, the side sheet 12 is folded inward in the width direction X. Next, the ventral region 51 of the main body portion 2 is folded toward the skin surface side Z1 so as to overlap the crotch region 53F, and thereafter, the crotch region 53F is folded toward the skin surface side Z1 so as to overlap the buttock region 53R. Further, the buttock region 53R is folded toward the skin surface side Z1 so as to overlap the dorsal region 52. By folding the diaper for pets 1 into four in this manner, the diaper for pets 1 is folded so that the ventral region 51 and the crotch region 53F are disposed inside and the dorsal region 52 and the buttock region 53R of the main body portion 2 are disposed outside.

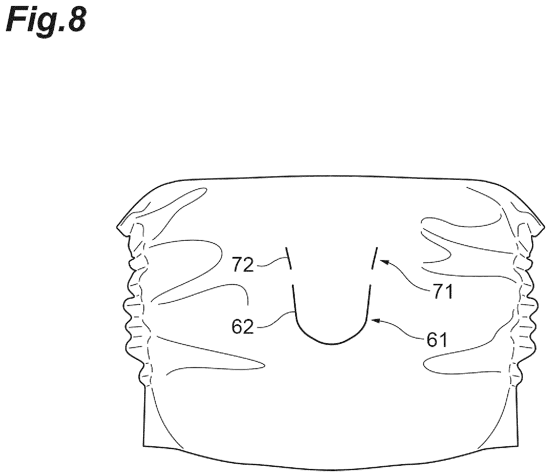

FIG. 8 is a plan view illustrating an example of the diaper for pets 1 folded in the above-described procedure. Since the first cut portion 61 and the second cut portion 71 are formed in the buttock region 53R, as illustrated in FIG. 8, the first cut portion 61 and the second cut portion 71 are disposed on the outer surface of the folded diaper for pets 1 by folding the diaper for pets 1 so that the buttock region 53R of the main body portion 2 is disposed outside. Note that in the embodiment illustrated in FIG. 8, the entire the first cut portion 61 and the second cut portion 71 are disposed on the outer surface of the diaper for pets 1, but only a part of the first cut portion 61 and the second cut portion 71 may be disposed on the outer surface of the folded diaper for pets 1.

As described above, since at least a part of the first cut portion 61 and the second cut portion 71 is disposed on the outer surface of the folded diaper for pets 1, the user can visually recognize the first cut portion 61 and the second cut portion 71 when taking out the folded diaper for pets 1 from the package 100. Therefore, it is possible to cause the user to recognize that it is possible to select whether to cause to defecate inside or outside of the diaper for pets 1. As long as at least a part of the first cut portion 61 and the second cut portion 71 is disposed on the outer surface of the folded diaper for pets 1, the folding method is not limited to the above-described example.

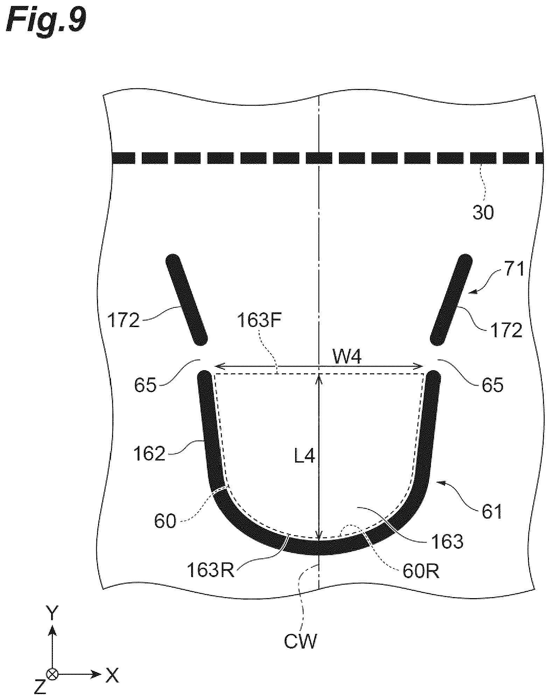

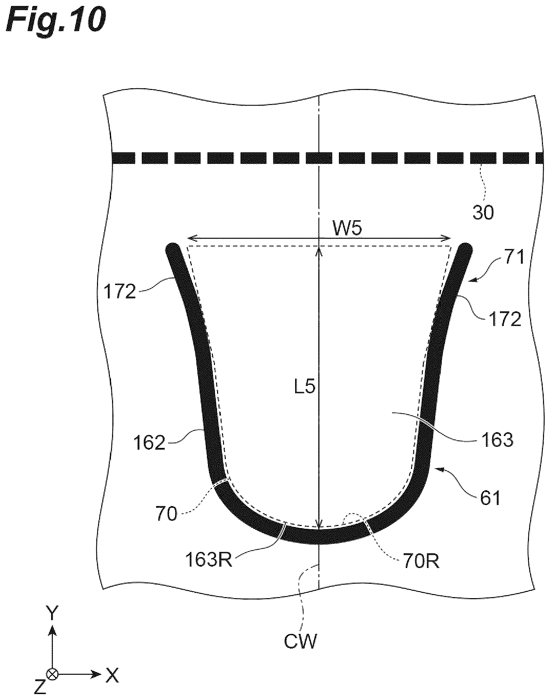

In the above-described embodiment, the second cut portion 71 is formed on the dorsal side of the main body portion 2 than the first cut portion 61, but the second cut portion 71 may be formed on the ventral side than the first cut portion 61. Hereinafter, a modification of the diaper for pets 1 will be described with reference to FIGS. 9 and 10.

As illustrated in FIG. 9, in the diaper for pets according to the modification, the first cut portion 61 is formed on the dorsal side of the main body portion 2 than the absorbent core 30, and the second cut portion 71 is formed between the first cut portion 61 and the absorbent core 30 in the longitudinal direction Y The non-cut portion 65 in which a slit is not formed is provided between the first cut portion 61 and the second cut portion 71, and the first cut portion 61 and the second cut portion 71 are spaced apart from each other via the non-cut portion 65.

As illustrated in FIG. 9, the first cut portion 61 includes a slit 162 having a substantially U-shape protruding toward the dorsal side when viewed from the skin surface side Z1. The main body portion 2 has a tongue piece 163 formed by making a substantially U-shaped cut in the main body portion 2 by the slit 162. The tongue piece 163 has a base end portion 163F coupled to the buttock region 53R of the main body portion 2 and a distal end portion 163R disposed on the opposite side of the base end portion 163F and separated from the buttock region 53R. The distal end portion 163R is directed to the dorsal side in a state where the tongue piece 163 is not folded back.

The tongue piece 163 is folded back toward the non-skin surface side Z2 starting from the base end portion 163F to open the first opening 60. Since the distal end portion 163R of the tongue piece 163 is directed to the dorsal side, when the tongue piece 163 is folded back toward the non-skin surface side Z2 starting from the base end portion 163F by passing the tail through the first opening 60, the tongue piece 63 abuts on the ventral surface side of the tail. At this time, the dorsal side end edge 60R of the first opening 60 abuts on the tail of the pet, whereby the diaper for pets 1 is positioned.

In one embodiment, as illustrated in FIG. 9, a maximum width W4 of the first opening 60 in the width direction X is larger than a maximum length L4 of the first opening 60 in the longitudinal direction Y That is, the first opening 60 has a laterally long shape that is long in the width direction X. Therefore, when the tail is passed through the first opening 60, an anus of the pet is not exposed from the first opening 60. As a result, when the pet defecates while wearing the diaper for pets 1 according to the modification, the feces do not pass through the first opening 60 and are excreted inside of the diaper for pets 1.

The second cut portion 71 includes a pair of slits 172. In the pair of slits 172, the pair of slits 172 are spaced apart from each other, and linearly extend so as to be separated from each other as approaching the absorbent core 30. The dorsal side end portions of the pair of slits 172 are disposed adjacent to the pair of end portions of the substantially U-shaped slit 62. The non-cut portion 65 is formed between the dorsal side end portions of the pair of slits 172 and the pair of end portions of the slit 162.

When the non-cut portion 65 is broken, as illustrated in FIG. 10, the slit 162 of the first cut portion 61 and the pair of slits 172 of the second cut portion 71 are coupled. The non-cut portion 65 is easily broken by pinching the tongue piece 163 with fingers from the non-skin surface side Z2 of the diaper for pets 1 and pulling the tongue piece to the ventral side. Accordingly, the tongue piece 163 is connected to a portion between the pair of slits 172 of the main body portion 2. In a state where the first cut portion 61 and the second cut portion 71 are connected, the tongue piece 163 is folded back toward the non-skin surface side Z2, whereby the second opening 70 is opened.

As illustrated in FIG. 10, the second opening 70 is an opening that encloses the first opening 60. That is, when the non-cut portion 65 is cut and the first cut portion 61 and the second cut portion 71 are coupled, the first opening 60 is enlarged to the ventral side to form the second opening 70. The second opening 70 is an opening having a semicircular shape or a tongue shape, and functions as an enlarged tail hole. The second opening 70 has a size capable of passing the feces of the pet. In one embodiment, the area of the second opening 70 may be twice or more the area of the first opening 60. In the embodiment illustrated in FIG. 10, an opening width of the second opening 70 in the width direction X increases toward the ventral side.

In order to pass the feces of the pet, the second opening 70 may have a vertically long shape that is long in the longitudinal direction Y For example, a maximum length L5 of the second opening 70 in the longitudinal direction Y may be larger than a maximum width W5 of the second opening 70 in the width direction X. In particular, the maximum length L5 of the second opening 70 may be twice or more the maximum length L4 of the first opening 60 in the longitudinal direction Y

As illustrated in FIG. 10, since the second opening 70 has a vertically long shape, when the tongue piece 163 is folded back toward the non-skin surface side Z2 by passing the tail through the second opening 70, an anus of the pet is exposed from the second opening 70. Therefore, when the pet defecates while wearing the diaper for pets 1, the feces pass through the second opening 70 and are excreted outside of the diaper for pets 1. In a case where the diaper for pets 1 is worn on the pet by passing the tail through the second opening 70, a dorsal side end edge 70R of the second opening 70 abuts on the tail of the pet.

As described above, the user of the diaper for pets 1 can select whether to cause to defecate inside or outside of the diaper for pets 1. Furthermore, in the embodiment illustrated in FIG. 10, since the tail hole of the diaper for pets 1 expands toward the ventral side when the second opening 70 is formed, the dorsal side end edge 70R of the second opening 70 and the dorsal side end edge 60R of the first opening 60 are disposed at the same position. Therefore, the position at which the tail of the pet abuts the diaper for pets 1 does not change between when the tail passes through the first opening 60 and when the tail passes through the second opening 70. Therefore, the diaper for pets 1 can be reliably fitted to the pet, and the displacement is less likely to occur in the diaper for pets 1. Therefore, urine leakage can be prevented.

Although the diapers for pets according to various embodiments have been described above, the present invention is not limited to the above-described embodiments, and various modifications can be made without changing the gist of the invention. That is, it should be noted that the above-described embodiments are for the purpose of illustration and are not intended to limit the scope of the present invention.

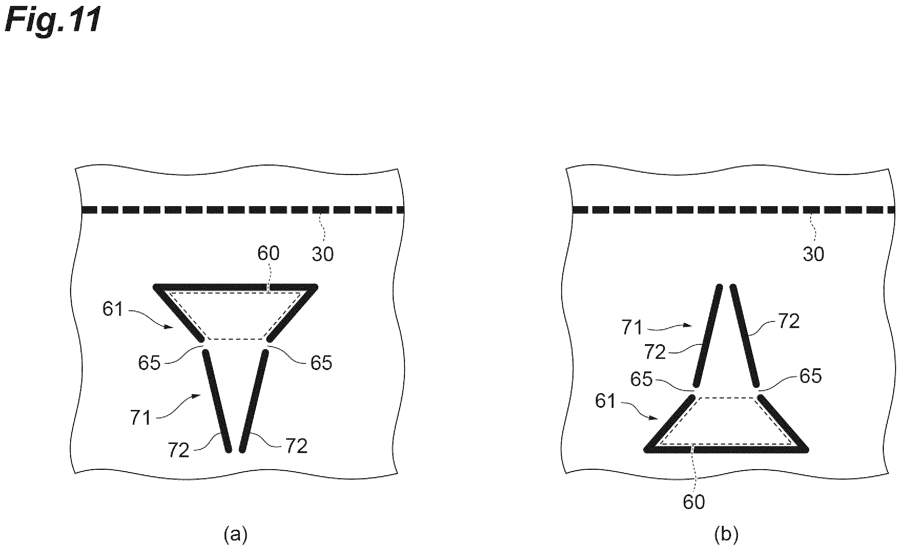

For example, the shape of the first opening 60 is not limited to a semicircular shape or a tongue shape. For example, as illustrated in FIGS. 11(a) and 11(b), the first opening 60 may have a trapezoidal shape. In the diaper for pets illustrated in FIG. 11(a), the first cut portion 61 forming the trapezoidal first opening 60 is disposed on the ventral side than the second cut portion 71. The pair of slits 72 of the second cut portion 71 linearly extend so as to approach each other as they separate from the absorbent core 30. In the diaper for pets illustrated in FIG. 11(b), the first cut portion 61 forming the trapezoidal first opening 60 is disposed on the dorsal side than the second cut portion 71. The pair of slits 72 of the second cut portion 71 linearly extend so as to approach each other as approaching the absorbent core 30. The first opening 60 may have a semicircular shape, a rectangular shape, a polygonal shape, or an elliptical shape.

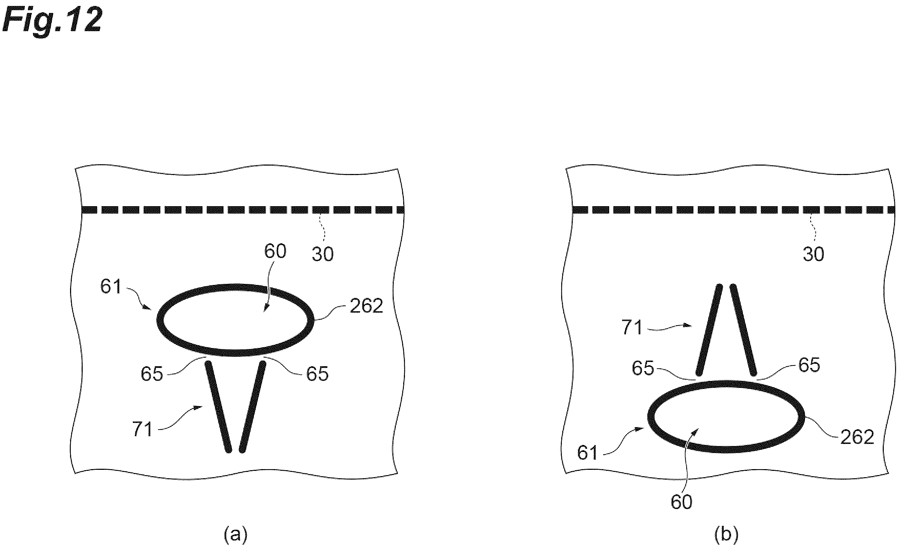

As illustrated in FIGS. 12(a) and 12(b), the first cut portion 61 may include an annular slit 262 extending over the entire periphery of the end edge of the first opening 60. By forming the annular slit 262, a part of the main body portion 2 is cut out, and the first opening 60 having no tongue piece is formed. Note that the annular slit 262 means a cut that cuts out a part of the main body portion 2 over the entire periphery, and a planar shape thereof is not limited to an annular shape, and includes an annular shape such as a rectangular annular shape and a polygonal annular shape. In the diaper for pets illustrated in FIG. 12(a), the first cut portion 61 including the annular slit 262 is disposed on the ventral side than the second cut portion 71. In the diaper for pets illustrated in FIG. 12(b), the first cut portion 61 including the annular slit 262 is disposed on the dorsal side than the second cut portion 71. In the embodiment illustrated in FIGS. 12(a) and 12(b), the first opening 60 having no tongue piece is formed. This allows the user to easily recognize the presence of the first opening 60.

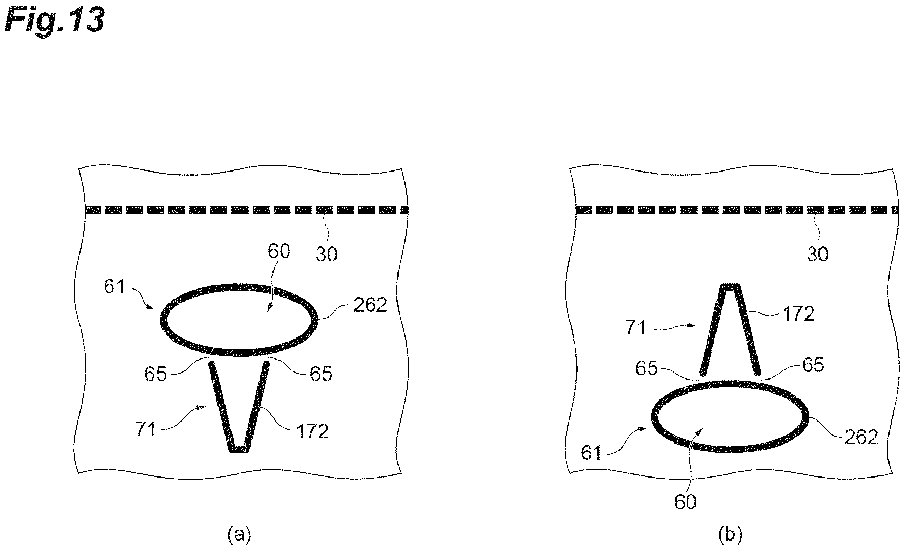

In the embodiment illustrated in FIG. 3, the second cut portion 71 includes the pair of linear slits 72, but the shape of the slit of the second cut portion 71 is not limited to a linear shape. For example, the second cut portion 71 may include a substantially V-shaped slit 172. In the diaper for pets illustrated in FIG. 13(a), the first cut portion 61 including the annular slit 262 and the second cut portion 71 disposed on the dorsal side than the first cut portion 61 and including the substantially V-shaped slit 172 protruding toward the dorsal side are formed in the main body portion 2. In the diaper for pets 1 illustrated in FIG. 13(b), the first cut portion 61 including the annular slit 262 and the second cut portion 71 disposed on the ventral side than the first cut portion 61 and including the substantially V-shaped slit 172 protruding toward the ventral side are formed in the main body portion 2. The shape of the slit of the second cut portion 71 may be substantially U-shaped or annular.

Similarly to the embodiment illustrated in FIG. 1, in the diapers for pets illustrated in FIGS. 11 to 13, in a case where the tail of the pet is inserted into the first opening 60 without breaking the non-cut portion 65, the feces of the pet are excreted inside of the diaper for pets 1, and in a case where the tail of the pet is inserted into the second opening 70 after breaking the non-cut portion 65, the feces of the pet are excreted outside of the diaper for pets 1. Therefore, the user of the diaper for pets 1 can select whether to discharge the feces of the pet to the inside or the outside of the diaper for pets 1.

The various embodiments described above can be combined within a consistent range.

### Reference Signs List

1 Diaper for pets
2 Main body portion
10 Front sheet
20 Back sheet
30 Absorbent core
40 Elastic member
45 Target portion
60 First opening
61 First cut portion
62, 72, 162, 172, 262 Slit
63, 163 Tongue piece
65 Non-cut portion
70 Second opening
71 Second cut portion
90 Fastening tab
100 Package
CL1 Center line
X Width direction
Y Longitudinal direction

## Claims

1. A diaper for pets accommodated in a package in a folded state, the diaper for pets comprising:
a main body portion having a longitudinal direction connecting a ventral side to a dorsal side of a pet and a width direction perpendicular to the longitudinal direction, the main body portion including a front sheet, a back sheet, and an absorbent core disposed between the front sheet and the back sheet, wherein
the main body portion is formed with a first cut portion to form a first opening through which a tail of the pet is capable of being inserted, and a second cut portion to form a second opening through which feces of the pet are capable of passing, the second opening having an area larger than an area of the first opening, and
at least a part of the first cut portion and the second cut portion is disposed on an outer surface of the folded main body portion.

2. The diaper for pets according to claim 1, wherein
the diaper for pets is folded in a state of being folded in three or four in the longitudinal direction, and
entire the first cut portion and the second cut portion are disposed on the outer surface of the folded main body portion.

3. The diaper for pets according to claim 1 or 2, wherein
the main body portion includes a non-cut portion disposed between the first cut portion and the second cut portion, and
the second cut portion forms the second opening enclosing the first opening together with the first cut portion when the non-cut portion is broken and the first cut portion and the second cut portion are coupled.

4. The diaper for pets according to claim 3, wherein
the first cut portion is formed on the dorsal side of the main body portion with respect to the absorbent core, and
the second cut portion is formed on the dorsal side of the main body portion with respect to the first cut portion.

5. The diaper for pets according to claim 4, wherein the first cut portion forms a flap-shaped tongue piece that is a part of the front sheet and the back sheet, and when the tongue piece is pulled to the dorsal side, the non-cut portion is broken and the first cut portion and the second cut portion are coupled.

6. The diaper for pets according to claim 5, wherein
the first cut portion includes a U-shaped slit protruding toward the ventral side, and
the second cut portion includes a pair of slits adjacent to a pair of end portions of the U-shaped slit via the non-cut portion.

7. The diaper for pets according to claim 6, wherein the pair of slits linearly extend so as to be separated from each other toward the dorsal side.

8. The diaper for pets according to claim 4, wherein the first cut portion includes an annular slit extending over an entire periphery of an end edge of the first opening.

9. The diaper for pets according to claim 3, wherein
the first cut portion is formed on the dorsal side of the main body portion with respect to the absorbent core, and
the second cut portion is formed between the first cut portion and the absorbent core in the longitudinal direction.

10. The diaper for pets according to claim 9, wherein the first cut portion forms a flap-shaped tongue piece that is a part of the front sheet and the back sheet, and when the tongue piece is pulled to the ventral side, the non-cut portion is broken and the first cut portion and the second cut portion are coupled.

11. The diaper for pets according to claim 10, wherein
the first cut portion includes a U-shaped slit protruding toward the dorsal side, and
the second cut portion includes a pair of slits adjacent to a pair of end portions of the U-shaped slit via the non-cut portion.

12. The diaper for pets according to claim 11, wherein the pair of slits linearly extend so as to be separated from each other toward the ventral side.

13. The diaper for pets according to claim 9, wherein the first cut portion includes an annular slit extending over an entire periphery of an end edge of the first opening.

14. The diaper for pets according to any one of claims 1 to 13, further comprising:
a pair of fastening tabs extending outward of the main body portion in the width direction; and
a target portion disposed on the dorsal side of the main body portion with respect to the pair of fastening tabs and engaged with the pair of fastening tabs, wherein
a dorsal side end edge of the second opening is disposed on the ventral side with respect to an intermediate line between a center line of the main body portion in the longitudinal direction and a ventral side end edge of the target portion.

15. The diaper for pets according to any one of claims 1 to 14, wherein an area of the second opening is twice or more an area of the first opening.

16. The diaper for pets according to any one of claims 1 to 15, wherein a maximum length of the second opening in the longitudinal direction is twice or more of a maximum length of the first opening in the longitudinal direction.

17. The diaper for pets according to any one of claims 1 to 16, wherein a maximum width of the first opening in the width direction is larger than the maximum length of the first opening in the longitudinal direction.

18. The diaper for pets according to any one of claims 1 to 17, wherein the maximum length of the second opening in the longitudinal direction is larger than a maximum width of the second opening in the width direction.

19. The diaper for pets according to any one of claims 1 to 18, further comprising an elastic member fixed to the main body portion in a state of being stretched in the longitudinal direction and extending so as to straddle the second opening in the longitudinal direction.

20. The diaper for pets according to any one of claims 1 to 19, wherein
a design is imparted to a surface of the back sheet, and
a design is not imparted to a surface of the front sheet.
